(19) Europäisches Patentamt
European Patent Office
Office européen des brevets

(11) Veröffentlichungsnummer: **0 418 716 B1**

(12) # EUROPÄISCHE PATENTSCHRIFT

(45) Veröffentlichungstag der Patentschrift: **06.04.94**

(51) Int. Cl.[5]: **C07D 451/10**, C07D 451/14, A61K 31/46, A61K 31/445

(21) Anmeldenummer: **90117554.7**

(22) Anmeldetag: **12.09.90**

Die Akte enthält technische Angaben, die nach dem Eingang der Anmeldung eingereicht wurden und die nicht in dieser Patentschrift enthalten sind.

(54) **Neue Thienylcarbonsäureester von Aminoalkoholen, ihre Ouaternierungsprodukte sowie die Herstellung und Verwendung dieser Verbindungen.**

(30) Priorität: **16.09.89 DE 3931041**

(43) Veröffentlichungstag der Anmeldung:
**27.03.91 Patentblatt 91/13**

(45) Bekanntmachung des Hinweises auf die Patenterteilung:
**06.04.94 Patentblatt 94/14**

(84) Benannte Vertragsstaaten:
**AT BE CH DE DK ES FR GB GR IT LI LU NL SE**

(56) Entgegenhaltungen:
**DE-B- 1 166 787**
**GB-A- 955 535**
**US-A- 3 673 195**
**US-A- 3 808 263**

**ACTA CHEMICA SCANDINAVICA, Band 24, 1970, Seiten 1590-1596, Copenhagen, DK; K. NYBERG et al.: "Investigation of dithienylglycolic esters"**

**J. Chem. Soc., 1957, S. 3575-3578**

(73) Patentinhaber: **BOEHRINGER INGELHEIM KG**
**D-55216 Ingelheim(DE)**

(84) Benannte Vertragsstaaten:
**BE CH DE DK ES FR GR IT LI LU NL SE AT**

(73) Patentinhaber: **BOEHRINGER INGELHEIM INTERNATIONAL G.M.B.H.**

**D-55216 Ingelheim(DE)**

(84) Benannte Vertragsstaaten:
**GB**

(72) Erfinder: **Banholzer, Rolf, Dr.**
**Johann-Calvin-Strasse 11**
**D-6507 Ingelheim am Rhein(DE)**
Erfinder: **Bauer, Rudolf, Dr.**
**Mainzerstr.58**
**D-6531 Ockenheim(DE)**
Erfinder: **Reichl, Richard, Dr.**
**Im Hippel 55**
**D-6535 Gau-Algesheim(DE)**

**EP 0 418 716 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue Thienylcarbonsäureester von Aminoalkoholen und ihre Quaternierungsprodukte sowie die Herstellung der neuen Verbindungen und ihre Verwendung als Wirkstoffe in Arzneimitteln.

Die neuen Verbindungen entsprechen der Formel

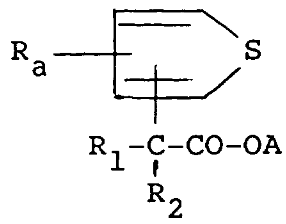

(I),

in der

A für die Gruppe

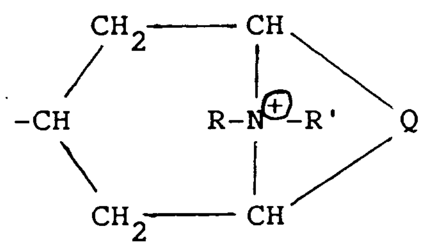

(II)

steht,

worin

Q eine der zweibindigen Gruppen

$-CH_2-CH_2-$, $-CH_2-CH_2-CH_2-$, $-CH=CH-$,

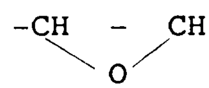

und R einen gegebenenfalls halogen- oder hydroxysubstituierten $C_1$-$C_4$-Alkylrest, R' einen $C_1$-$C_4$-Alkylrest bedeutet und R und R' gemeinsam auch einen $C_4$-$C_6$-Alkylenrest bilden können, und der positiven Ladung des N-Atoms ein Äquivalent eines Anions gegenübersteht ($X^{\ominus}$),

$R_1$ für einen Thienyl-, Phenyl-, Furyl-, Cyclopentyl- oder Cyclohexylrest, wobei diese Reste auch methylsubstituiert, Thienyl- und Phenyl auch fluor- oder chlorsubstituiert sein können,

$R_2$ für Wasserstoff, OH oder $C_1$-$C_4$-Alkoxy oder $C_1$-$C_4$-Alkyl,

$R_a$ für H, F, Cl oder $CH_3$ steht.

In den Verbindungen der Formel I steht $R_1$ bevorzugt für Thienyl, $R_2$ bevorzugt für OH. Die Gruppe -OA hat bevorzugt $\alpha$-Konfiguration und leitet sich z.B. von Scopin, Tropin, Granatolin oder 6,7-Dehydrotropin bzw. entsprechenden Nor-Verbindungen ab; -OA kann jedoch auch $\beta$-Konfiguration aufweisen wie in Pseudotropin, Pseudoscopin.

Entsprechende Reste sind z.B.

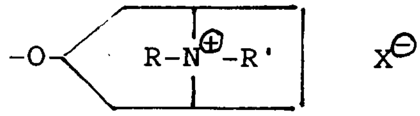

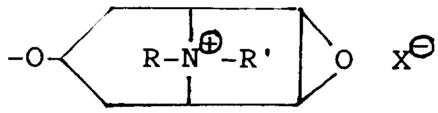

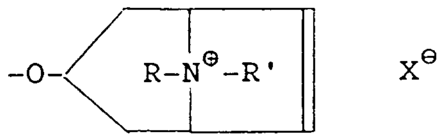

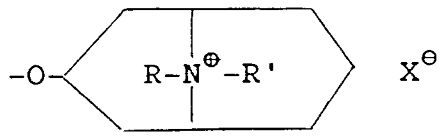

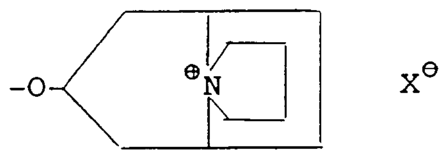

Der Substituent R ist bevorzugt ein niederer Alkylrest wie $CH_3$, $C_2H_5$, n-$C_3H_7$, i-$C_3H_7$, R' bevorzugt $CH_3$. R und R' gemeinsam sind z.B. $-(CH_2)_5-$. Als Halogensubstituent für R kommt F oder in zweiter Linie Cl in Frage.
Bedeutet R einen halogen- oder hydroxysubstituierten Alkylrest, so handelt es sich bevorzugt um $-CH_2-CH_2F$ bzw. $-CH_2-CH_2OH$. Dementsprechend steht die Gruppe A z.B. für die Reste des Scopins, des N-Ethylnorscopins, des N-Isopropylnorscopins, des Tropins, des N-Isopropylnortropins, des 6,7-Dehydrotropins, des N-$\beta$-Fluorethylnortropins, des N-Isopropyl-6,7-Dehydronortropins, des N-Methylgranatolins als die entsprechenden Quartärverbindungen, wobei das Anion bevorzugt $Br^{\ominus}$ bzw. $CH_3SO_3^{\ominus}$ ist.

Als Säurerest

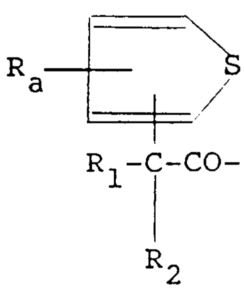

(III)

kommen vor allem in Betracht:

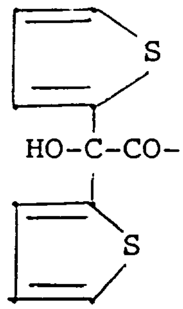

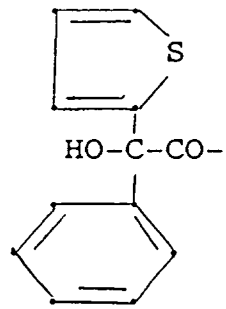

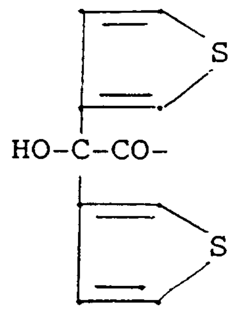

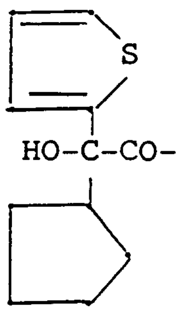

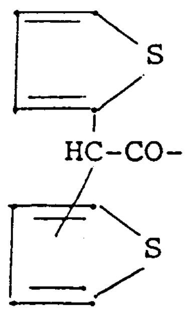

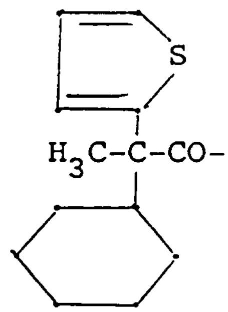

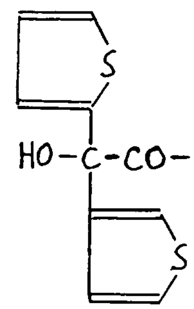

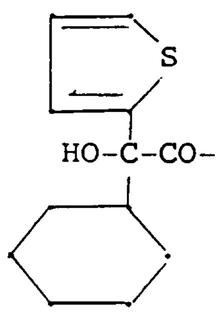

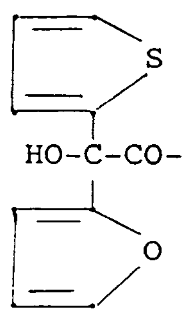

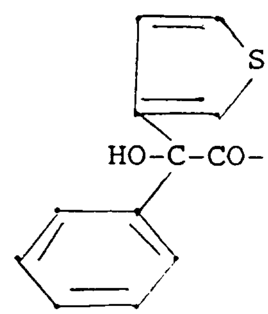

Die erfindungsgemäßen Verbindungen stellen stark und lange wirksame Anticholinergika dar. Bei Dosierungen im µg-Bereich werden inhalativ Wirkungsdauern von mindestens 24 Stunden erreicht. Die Toxizität liegt zudem im gleichen Bereich wie bei dem Handelsprodukt Ipratropiumbromid, während gleichzeitig die therapeutische Wirkung stärker ist.

Die neuen Verbindungen eignen sich, entsprechend ihrer Natur als Anticholinergika, z.B zur Behandlung von chronisch obstruktiver Bronchitis und (leichtem bis mittelschwerem) Asthma, ferner zur Behandlung vagal bedingter Sinusbradykardien.

Während sich bei Atemwegserkrankungen hauptsächlich die inhalative Anwendung der neuen Wirkstoffe (insbesondere der Quartärverbindungen) empfiehlt, wodurch Nebenwirkungen weitgehend ausgeschaltet werden, erfolgt die Anwendung bei Sinusbradykardien vorzugsweise intravenös oder oral. Dabei erweist sich als vorteilhaft, daß die neuen Verbindungen die Magen/Darm-Motilität weitgehend unbeeinflußt lassen.

Aus Acta Chem. Scand, Bd. 24 (1970) Seiten 1590-6 ist die Herstellung von 3-Tropanyl-2,2'- bzw. 3,3'-dithienylglykolat bekannt. Über physiologische Eigenschaften dieser tertiären Verbindungen wird nichts gesagt. Der Tropinester der Phenyl-2-thienylglykolsäure ist aus J. Chem. Soc. (1957), Seiten 3575-8 bekannt. Dort ist auch die spasmolytische Wirkung der Verbindung am isolierten Kaninchendarm erwähnt. Ein Hinweis auf eine überraschend starke und langanhaltende Wirkung als Broncholytikum durch die Überführung der Verbindung in entsprechende Quartärverbindungen findet sich nicht.

N-substituierte Norgranatanol-3β-ester beschreibt GB 955 535. Diese Verbindungen sind gemäß der Patentschrift als hervorragende zentral wirkende Anticholinergika zu charakterisieren. Die erfindungsgemäßen vor allem anhaltend broncholytisch wirkenden Verbindungen werden durch die GB-Patentschrift nicht

nahegelegt.

Für die Applikation werden die erfindungsgemäßen Verbindungen mit bekannten Hilfs- und/oder Trägerstoffen zu gebräuchlichen galenischen Zubereitungen verarbeitet, z.B. zu Inhalationslösungen, Suspensionen in verflüssigten Treibgasen, Liposomen bzw. Proliposomen enthaltenden Zubereitungen, Injektionslösungen, Tabletten, Dragrées, Kapseln, Inhalationspulvern zur Anwendung in üblichen Inhalationsgeräten.

Formulierungsbeispiele (Angaben in Gewichtsprozent):

1. Dosieraerosol

| | |
|---|---|
| Wirkstoff gemäß der Erfindung | 0,005 |
| Sorbitantrioleat | 0,1 |
| Monofluortrichlormethan und Difluordichlormethan 2 : 3 | ad 100 |

Die Suspension wird in einen üblichen Aerosolbehälter mit Dosierventil gefüllt. Pro Betätigung werden vorzugsweise 50 µl Suspension abgegeben. Der Wirkstoff kann gewünschtenfalls auch höher dosiert werden (z.B. 0.02 Gew.-%).

2. Tabletten

| | |
|---|---|
| Wirkstoff gemäß der Erfindung | 0,05 |
| Kolloidale Kieselsäure | 0,95 |
| Milchzucker | 65,00 |
| Kartoffelstärke | 28,00 |
| Polyvinylpyrrolidon | 3,00 |
| Na-Celluloseglykolat | 2,00 |
| Magnesiumstearat | 1,00 |

Die Bestandteile werden in üblicher Weise zu Tabletten von 200 mg verarbeitet.

Die vorteilhaften Eigenschaften der neuen Verbindungen zeigen sich beispielsweise in der Hemmung der Broncholyse am Kaninchen (Acetylcholinspasmus i.v.). Nach intravenöser Gabe der neuen Wirkstoffe (Dosis 3 µg/kg i.v.) trat die maximale Wirkung nach 10 bis 40 Minuten ein. Nach 5 Stunden war die Hemmwirkung noch nicht auf die Hälfte gesunken, d.h. die Halbwirkungszeit beträgt mehr, z.T. erheblich mehr als 5 Stunden, wie die nachstehend aufgeführten Restwirkungen nach 5 Stunden deutlich machen:

| Verbindung | Restwirkung in % |
|---|---|
| A | 76 |
| B | 76 |
| C | 81 |
| D | 61 |
| E | 68 |
| F | 73 |
| G | 69 |

Verbindungen der Formel

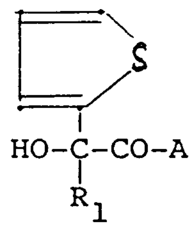

| Verbindung | A | R1 |
|---|---|---|
| A | 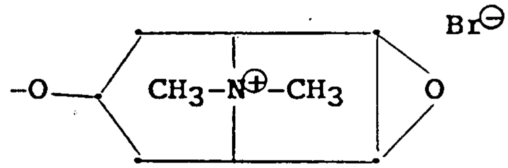 | 2-Thienyl |
| B | 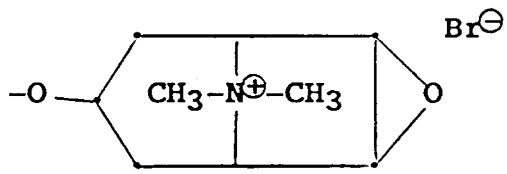 | 3-Thienyl |
| D | 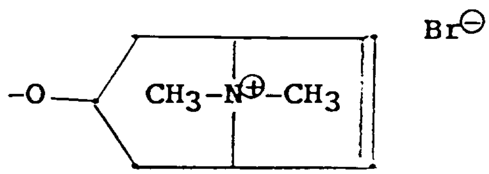 | 2-Thienyl |
| E | 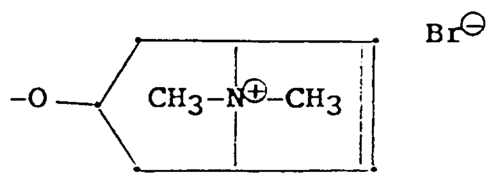 | 3-Thienyl |
| F | 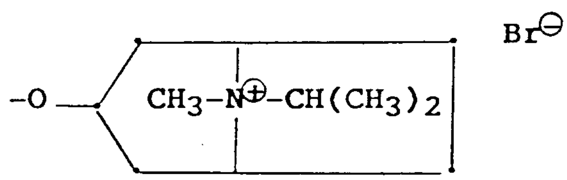 | Cyclopentyl |
| G | 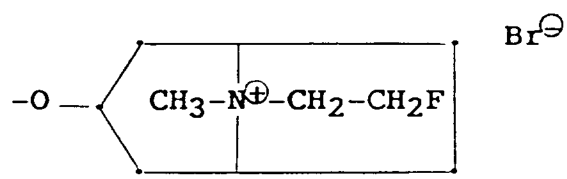 | Cyclopentyl |

Verbindung C

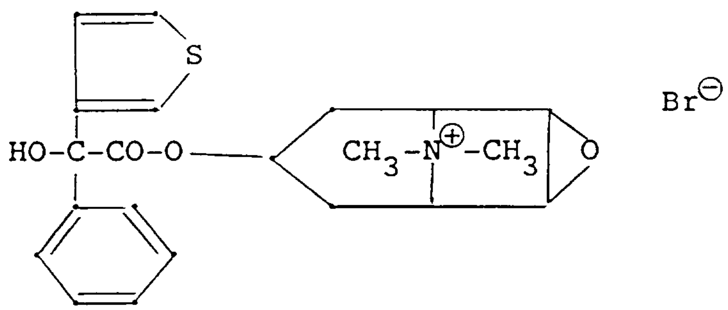

Anmerkungen:

1. Bei den Verbindungen, in denen $R_1$ nicht 2-Thienyl ist, handelt es sich um Racemate.
2. Es handelt sich jeweils um 3α-Verbindungen.

Zur Herstellung der neuen Verbindungen dienen an sich bekannte Verfahren. Bevorzugt wird ein Ester der Formel

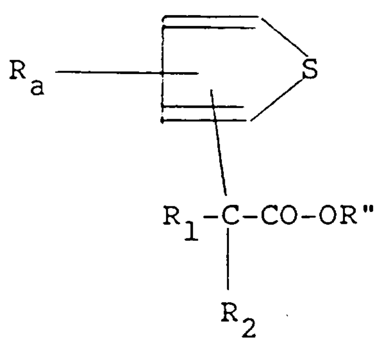

(IV),

worin R" für einen $C_1$-$C_4$-Alkylrest, vorzugsweise für einen Methyl oder Ethylrest steht ($R_1$, $R_2$ und $R_a$ haben die obige Bedeutung), mit einem Aminoalkohol der Formel

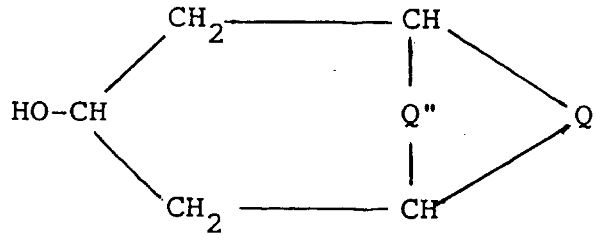

(V)

worin Q die obige Bedeutung hat, Q" für -NR oder für -NH steht und die OH-Gruppe sich in α- oder β-Stellung befindet, in Gegenwart eines üblichen Umesterungskatalysators umestert und die erhaltene Verbindung

a) wenn Q" -NR bedeutet, mit einem reaktionsfähigen Monoderivat Z-($C_1$-$C_4$-Alkyl) eines entsprechenden Alkans (Z = Abgangsgruppe) quaterniert

oder

b) wenn Q" = NH bedeutet, mit einem endständig disubstituierten Alkan Z-($C_4$-$C_6$-Alkylen)-Z ohne Zwischenisolierung quaterniert.

Die Umesterung wird in der Wärme in einem organischen Lösungsmittel, z.B. Toluol, Xylol, Heptan, oder in der Schmelze durchgeführt, wobei starke Basen wie Natriummethylat, Natriumethylat, Natriumhydrid, metallisches Natrium, als Katalysator dienen. Zur Entfernung des freigesetzten niederen Alkohols aus dem Gleichgewicht wird verminderter Druck angewendet, ggf. der Alkohol azeotrop abdestilliert. Die Umesterung erfolgt bei Temperaturen, die im allgemeinen 95 °C nicht überschreiten. Häufig verläuft die Umesterung in der Schmelze günstiger.
Aus Säureadditionssalzen der tertiären Amine kann man gewünschtenfalls mit geeigneten basischen Verbindungen in an sich bekannter Weise die freien Basen erhalten. Die Quaternierung wird in geeigneten Lösungsmitteln, etwa Acetonitril oder Acetonitril/Methylenchlorid vorzugsweise bei Raumtemperatur durchgeführt; dabei wird als Quaternierungsreagenz bevorzugt ein entsprechendes Alkylhalogenid, z.B. Alkylbromid, verwendet. Umesterungsprodukte mit Q'' in der Bedeutung NH dienen als Ausgangsstoffe für diejenigen Verbindungen, in denen R und R' gemeinsam eine $C_4$-$C_6$-Alkylengruppe darstellen. Die Überführung in die tertiäre und dann quartäre Verbindung erfolgt dann mit Hilfe geeigneter 1,4-, 1,5- bzw. 1,6-Dihalogenalkane ohne Zwischenisolierung.

Die Ausgangsstoffe können - soweit sie nicht schon beschrieben wurden - analog zu bekannten Verbindungen erhalten werden.

Beispiele:

Di-(2-thienyl)glykolsäuremethylester aus Oxalsäuredimethylester und 2-Thienylmagnesiumbromid;
Di-(2-thienyl)glykolsäureethylester aus (2-Thienyl)glyoxylsäure und 2-Thienyllithium;
Hydroxy-phenyl-(2-thienyl)essigsäureethylester aus Phenylglyoxylsäuremethylester und 2-Thienylmagnesiumbromid oder aus (2-Thienyl)glyoxylsäuremethylester und Phenylmagnesiumbromid.
Ähnlich können 2-Thienylglyoxylsäuremethylester und Cyclohexyl- bzw. Cylopentylmagnesiumbromid umgesetzt werden.

Auch für die Herstellung der Aminoalkohole stehen mehrere Verfahren zur Verfügung.

Pseudoscopin kann nach M. Polonovski et al., Bull. soc. chim. <u>43</u>, 79 (1928) erhalten werden.

Pseudotropenol kann aus dem Gemisch isoliert werden (fraktionierte Kristallisation bzw. Destillation), das z.B. nach V. Hayakawa et al., J. Amer.Chem.Soc. <u>1978</u>, 100(6), 1786 bzw. R. Noyori et al., J.Amer.Chem.Soc. <u>1974</u>, 96(10), 3336 erhalten wird.

Ausgehend von 2- bzw. 3-Furylglyoxylnitril können über die daraus erhältliche 2- bzw. 3-Furylglyoxylsäure die entsprechenden Methylester auf übliche Weise hergestellt werden. Aus diesen werden wie beschrieben mit den metallorganischen Derivaten von 2-bzw. 3-Bromthiophen die entsprechenden Glykolsäureester erhalten. Die aus 2-, 3- oder 4-Halogenpyridin erhältlichen metallorganischen Verbindungen lassen sich mit 2- bzw. 3-Thienylglyoxylsäuremethylester zu den entsprechenden Glykolsäureestern umsetzen.

Thienylglykolsäureester, in denen der Thiophenring in 2- bzw. 3-Stellung Fluor enthält, werden z.B. ausgehend von 2-Fluor- bzw. 3-Fluorthiophen hergestellt (Bromieren zu 2-Brom-3-fluor- oder 2-Brom-5-fluorthiophen und, nach Überführung in entsprechende metallorganische Verbindungen, Umsetzung mit geeigneten Glyoxylsäureestern zu den Glykolsäureestern.

2-Fluorthiophen und 3-Fluorthiophen lassen sich analog Unterhalt, Arch.Pharm. <u>322</u>, 839 (1989) zu den entsprechenden Glyoxylsäureestern umsetzen, die ihrerseits, wie schon beschrieben, mit z.B. 2- oder 3-Thienylderivaten, zu Glykolsäureestern umgesetzt werden können. Durch geeignete Auswahl der Komponenten lassen sich analog symmetrisch substituierte Di-thienylglykolsäureester herstellen.

Ein weiterer Weg bietet sich an analog zur Benzoinkondensation und Benzilsäureumlagerung.

Die folgenden Beispiele erläutern die Erfindung, ohne sie zu beschränken.

<u>Beispiel 1</u>

<u>Di-(2-thienyl)glykolsäurescopinester</u>

50,87 g (0,2 mol) Di-(2-thienyl)glykolsäuremethylester und 31,04 g (0,2 mol) Scopin werden in 100 ml abs. Toluol gelöst und unter Zugabe von 1,65 g (0,071 Grammatome) Natrium in mehreren Anteilen bei einer Badtemperatur von 90 °C umgesetzt. Bei einer Temperatur von 78 - 90 °C des Reaktionsgemisches wird unter einem Druck von 500 mbar das entstehende Methanol abdestilliert. Nach einer Reaktionszeit von ca. 5 Stunden wird das Reaktionsgemisch in eine Mischung aus Eis und Salzsäure gerührt. Die saure Phase wird abgetrennt, mit Natriumcarbonat alkalisch gemacht und die freie Base mit Methylenchlorid extrahiert. Nach dem Trocknen über Natriumsulfat wird das Methylenchlorid unter vermindertem Druck abdestilliert und der Rückstand aus Acetonitril umkristallisiert;

beigefarbene Kristalle (aus Acetonitril),
Fp. 149 - 50 °C,
Ausbeute: 33,79 g (44,7 % d. Th.).

Beispiel 2

Di-(2-thienyl)glykolsäurescopinester

12,72 g (0,05 mol) Di-(2-thienyl)glykolsäuremethylester und 7,76 g (0,05 mol) Scopin werden in einem Heizbad von 70 °C unter Wasserstrahlvakuum geschmolzen. In diese Schmelze werden 2,70 g (0,05 mol) Natriummethylat eingetragen und unter Wasserstrahlvakuum 1 Stunde in einem Heizbad von 70 °C und zur Nachreaktion eine weitere Stunde in einem Heizbad von 90 °C erhitzt. Die erstarrte Schmelze wird in einem Gemisch von 100 ml Wasser und 100 ml Methylenchlorid unter Temperaturkontrolle aufgenommen und die Methylenchloridphase mehrmals mit Wasser extrahiert. Die Methylenchloridphase wird mit der entsprechenden Menge verdünnter Salzsäure extrahiert. Aus den gesammelten Wasserphasen wird nach Zugabe der entsprechenden Menge Natriumcarbonat der Di-(2-thienyl)glykolsäurescopinester mit Methylenchlorid extrahiert und über Natriumsulfat getrocknet. Aus der getrockneten Methylenchloridlösung wird auf übliche Art und Weise das Hydrochlorid hergestellt. Die Kristalle werden abgesaugt, mit Aceton gewaschen und bei 35 °C unter vermindertem Druck getrocknet. Leicht gelbe Kristalle (aus Methanol), Fp. 238 - 41 °C (Zers.);
Ausbeute: 10,99 g (53,1 % d.Th.).
Das Hydrochlorid kann auf übliche Art und Weise in die Base überführt werden.

Beispiel 3

Di-(2-thienyl)glykolsäurescopinester

38,15 g (0,15 mol) Di-(2-thienyl)glykolsäuremethylester und 23,28 g (0,15 mol) Scopin werden vermischt, 0,34 g (0,015 Grammatome) Natrium zugegeben und unter Wasserstrahlvakuum in einem Heizbad von 90 °C geschmolzen. Die Umsetzungsdauer beträgt 2,5 Stunden. Danach werden 100 ml abs. Toluol zugegeben und solange bei einer Heizbadtemperatur von 90 °C gerührt, bis eine Lösung entsteht. Die Reaktionslösung wird auf Raumtemperatur abgekühlt und in eine mit Eis gekühlte Mischung aus Eis und Salzsäure gerührt. Das auskristallisierende Hydrochlorid des basischen Esters wird abgesaugt, mit wenig Wasser und ausgiebig mit Diethylether gewaschen. Die Phasen des Filtrats werden abgetrennt und die wäßrige Phase mit Diethylether extrahiert. Das abgesaugte Hydrochlorid wird in der (sauren) Wasserphase suspendiert und unter Temperaturkontrolle und unter Zugabe der entsprechenden Menge Natriumcarbonat in die Base überführt, die mit Methylenchlorid extrahiert wird. Die gesammelten Methylenchloridphasen werden über Natriumsulfat getrocknet. Nach dem Abdestillieren des Methylenchlorids hinterbleibt ein Kristallisat, das über Aktivkohle gereinigt aus Acetonitril umkristallisiert wird. Leicht gelbe Kristalle (aus Acetonitril), Fp. 148 - 49 °C;
Ausbeute: 39,71 g (70,1 % d.Th.).

Tabelle I

Verbindungen der Formel

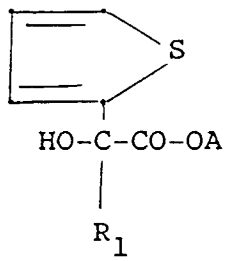

| Nr. | A | $R_1$ | Base | Fp. [°C] Hydrochlorid |
|---|---|---|---|---|
| 1 | 3α-(6ß,7ß-Epoxy)-tropanyl | 2-Thienyl | 149-50 | 238-41 |
| 2 | 3α-Tropanyl | 2-Thienyl | 167-8 | 253 |
| 3 | 3α-(6,7-Dehydro)-tropanyl | 2-Thienyl | 164-5 | |
| 4 | 3α-(N-ß-Fluorethyl)-nortropanyl | 2-Thienyl | | 236 |
| 5 | 3α-(N-Isopropyl)-granatanyl | 2-Thienyl | | 232 |
| 6 | 3α-(N-Isopropyl)-nortropanyl | 2-Thienyl | | 250 |
| 7 | 3α-(6ß,7ß-Epoxy)-N-isopropylnortropanyl | 2-Thienyl | | 206 |
| 8 | 3α-(6ß,7ß-Epoxy)-N-ethylnortropanyl | 2-Thienyl | | 212-3 |
| 9 | 3α-(N-Ethyl)-nortropanyl | 2-Thienyl | | 256-7 |
| 10 | 3α-(N-N-Methyl)-granatanyl | 2-Thienyl | | 241 |
| 11 | 3α-(6ß,7ß-Epoxy)-N-ß-fluorethylnortropanyl | 2-Thienyl | | 188-90 |
| 12 | 3α-(6ß,7ß-Epoxy)-N-n-propylnortropanyl | 2-Thienyl | 104-6 | |
| 13 | 3α-(6ß,7ß-Epoxy)-N-n-butylnortropanyl | 2-Thienyl | | 225-7 |
| 14 | 3α-(6ß,7ß-Epoxy)-tropanyl | Phenyl | | 246-7 |

| Nr. | A | $R_1$ | Base | Hydro-chlorid |
|---|---|---|---|---|
| 15 | 3α-Tropanyl | Phenyl | | 243-4 |
| 16 | 3α-(N-ß-Fluorethyl)-nortropanyl | Phenyl | | 219-20 |
| 17 | 3α-(6,7-Dehydro)-tropanyl | Phenyl | 181-3 | |
| 18 | 3α-(N-Ethyl)-nortropanyl | Phenyl | | 231-2 |
| 19 | 3α-(N-Isopropyl)-nortropanyl | Phenyl | | 246-7 |
| 20 | 3α-Tropanyl | Cyclohexyl | | 260 |
| 21 | 3α-(N-ß-Fluorethyl)-nortropanyl | Cyclohexyl | | 203-4 |
| 22 | 3α-(6ß,7ß-Epoxy)-tropanyl | Cyclopentyl | | 237 |
| 23 | 3α-Tropanyl | Cyclopentyl | | 260 |
| 24 | 3α-(N-ß-Fluorethyl)-nortropanyl | Cyclopentyl | | 182-3 |
| 25 | 3α-(N-Ethyl)-nortropanyl | Cyclopentyl | | 227-8 |
| 26 | 3α-(N-Isopropyl)-nortropanyl | Cyclopentyl | | 174-5 |
| 27 | 3ß-(6ß,7ß-Epoxy)-tropanyl | 2-Thienyl | | 240-2 |
| 28 | 3ß-Tropanyl | 2-Thienyl | | 217-9 |
| 29 | 3ß-(6,7-Dehydro)-tropanyl | 2-Thienyl | | 233-5 |
| 30 | 3α-(6,7-Dehydro)-tropanyl | 3-Thienyl | | 247-8 |
| 31 | 3α-(6ß,7ß-Epoxy)-tropanyl | 3-Thienyl | | 242-3 |
| 32 | 3α-(6ß,7ß-Epoxy)-tropanyl | 2-Furyl | | |
| 33 | 3α-(6,7-Dehydro)-tropanyl | 2-Furyl | | |
| 34 | 3α-Tropanyl | 2-Furyl | | |
| 35 | 3α-Tropanyl | 2-Pyridyl | | |
| 36 | 3α-(6ß,7ß-Epoxy)-tropanyl | 2-Pyridyl | | |
| 37 | 3α-(6,7-Dehydro)-tropanyl | 2-Pyridyl | | |
| 38 | 3α-Tropanyl | 3-Thienyl | | |
| 39 | 3α-(6,7-Dehydro)-tropanyl | Cyclopentyl | | |
| 40 | 3α-(6ß,7ß-Epoxy)-tropanyl | Cyclohexyl | | |
| 41 | 3α-(6,7-Dehydro)-tropanyl | Cyclohexyl | | |

Anmerkung: Alle Hydrochloride schmelzen unter Zersetzung.

Beispiel 4

Di-(2-thienyl)glykolsäurescopinester-methobromid

10,0 g (0,0265 mol) Di-(2-thienyl)glykolsäurescopinester werden in einem Gemisch, bestehend aus 20 ml wasserfreiem Methylenchlorid und 30 ml wasserfreiem Acetonitril gelöst und mit 12,8 g (0,1325 mol) Methylbromid (als 50 %ige Lösung in wasserfreiem Acetonitril) versetzt und das Reaktionsgemisch in einem Reaktionsgefäß dicht verschlossen bei Raumtemperatur 24 Stunden stehen gelassen. Während dieser Zeit fallen Kristalle aus. Diese werden abgesaugt, mit Methylenchlorid gewaschen und bei 35 °C unter vermindertem Druck getrocknet.
Weiße Kristalle (aus Methanol/Aceton), Fp. 217 - 8 °C (Zers.) nach dem Trocknen bei 111 °C unter vermindertem Druck.

Tabelle II

Quartärverbindungen der Formel

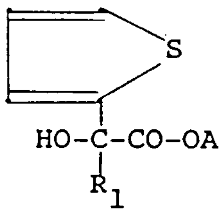

| Nr. | A | $R_1$ | Fp.[°C] |
|---|---|---|---|
| 1 | 3α-(6ß,7ß-Epoxy)-tropanyl-methobromid | 2-Thienyl | 217-18 |
| 2 | 3α-Tropanyl-methobromid | 2-Thienyl | 263-64 |
| 3 | 3α-(6,7-Dehydro)-tropanyl-methobromid | 2-Thienyl | 191-92 |
| 4 | 3α-(N-ß-Fluorethyl)-nortropanyl-methobromid | 2-Thienyl | 242-43 |
| 5 | 3α-Tropanyl-ß-fluorethobromid | 2-Thienyl | 214-15 |
| 6 | 3α-(N-Isopropyl)-granatanyl-methobromid | 2-Thienyl | 229-30 |
| 7 | 3α-(N-Isopropyl)-nortropanyl-methobromid | 2-Thienyl | 245-46 |
| 8 | 3α-(6ß,7ß-Epoxy)-N-isopropyl-nortropanyl-methobromid | 2-Thienyl | 223-24 |
| 9 | 3α-(6ß,7ß-Epoxy)-N-ethylnor-tropanyl-methobromid | 2-Thienyl | 215-16 |
| 10 | 3α-(N-Ethyl)-nortropanyl-methobromid | 2-Thienyl | 260-61 |
| 11 | 3α-(N-Methyl)-granatanyl-methobromid | 2-Thienyl | 246-47 |

| Nr. | A | $R_1$ | Fp.[°C] |
|---|---|---|---|
| 12 | 3α-(6ß,7ß-Epoxy)-N-ß-fluor-ethyl -nortropanyl-methobromid | 2-Thienyl | 182-83 |
| 13 | 3α-(6ß,7ß-Epoxy)-N-n-propyl-nortropanyl-methobromid | 2-Thienyl | 209-10 |
| 14 | 3α-Tropanyl-ß-hydroxyethobromid | 2-Thienyl | 231-32 |
| 15 | 3α-(6ß,7ß-Epoxy)-tropanyl-methobromid | Phenyl | 217-18 |
| 16 | 3α-Tropanyl-methobromid | Phenyl | 273-74 |
| 17 | 3α-(N-ß-Fluorethyl)-nortropanyl-methobromid | Phenyl | 215 |
| 18 | 3α-(6,7-Dehydro)-tropanyl-methobromid | Phenyl | 170-71 |
| 19 | 3α-(N-Ethyl)-nortropanyl-methobromid | Phenyl | 249-50 |
| 20 | 3α-(N-Isopropyl)-nortropanyl-methobromid | Phenyl | 259-60 |
| 21 | 3α-Tropanyl-ethobromid | Phenyl | 248-49 |
| 22 | 3α-(N-Ethyl)-nortropanyl-ethobromid | Phenyl | 244-45 |
| 23 | 3α-(6ß,7ß-Epoxy)-tropanyl-ethobromid | Phenyl | 226 |
| 24 | 3α-Tropanyl-ß-fluorthobromid | Phenyl | 241 |
| 25 | 3α-Tropanyl-methobromid | Cyclohexyl | 278 |
| 26 | 3α-(N-ß-Fluorethyl)-nortropanyl-methobromid | Cyclohexyl | 198 |
| 27 | 3α-Tropanyl-ß-fluorethobromid | Cyclohexyl | 233-34 |
| 28 | 3α-Tropanyl-methobromid | Cyclopentyl | 260 |
| 29 | 3α-Tropanyl-ethobromid | Cyclopentyl | 235-36 |
| 30 | 3α-(N-Ethyl)-nortropanyl-methobromid | Cyclopentyl | 251-52 |
| 31 | 3α-(N-Isopropyl)-nortropanyl-methobromid | Cyclopentyl | 244-45 |

| Nr. | A | $R_1$ | Fp.[°C] |
|---|---|---|---|
| 32 | 3α-Tropanyl-ß-fluorethobromid | Cyclopentyl | 189-90 |
| 33 | 3α-(N-ß-Fluorethyl)-nortropanyl-methobromid | Cyclopentyl | 226-27 |
| 34 | 3α(6,7-Dehydro)-tropanyl-metho-methansulfonat | 2-Thienyl | 225-6 |
| 35 | 3ß-(6ß,7ß-Epoxy)-tropanyl-methobromid | 2-Thienyl | 218-20 |
| 36 | 3ß-Tropanyl-methobromid | 2-Thienyl | 243-4 |
| 37 | 3ß-(6,7-Dehydro)-tropanyl-methobromid | 2-Thienyl | 211-4 |
| 38 | 3α(6,7-Dehydro)-tropanyl-methobromid | 3-Thienyl | 182-3* |
| 39 | 3α-(6ß,7ß-Epoxy)-tropanyl-methobromid | 3-Thienyl | 217-8 |
| 40 | (+)-Enantiomeres von Nr. 1 | | |
| 41 | (-)-Enantiomeres von Nr. 1 | | |
| 42 | 3α-(6ß,7ß-Epoxy)-tropanyl-methobromid | 2-Furyl | |
| 43 | 3α-(6,7-Dehydro)-tropanyl-methobromid | 2-Furyl | |
| 44 | 3α-Tropanyl-methobromid | 2-Furyl | |
| 45 | 3α-(6ß,7ß-Epoxy)-tropanyl methobromid | 2-Pyridyl | |
| 46 | 3α-(6,7-Dehydro)-tropanyl methobromid | 2-Pyridyl | |
| 47 | 3α-Tropanyl-methobromid | 2-Pyridyl | |
| 48 | 3α-Tropanyl-methobromid | 3-Thienyl | |
| 49 | 3α-(6,7-Dehydro)-tropanyl methobromid | Cyclopentyl | |

* enthält Kristallmethanol

| Nr. | A | $R_1$ | Fp.[°C] |
|---|---|---|---|
| 50 | 3α-(6ß,7ß-Epoxy)-tropanyl methobromid | Cyclohexyl | |
| 51 | 3α-(6,7-Dehydro)-tropanyl methobromid | Cyclohexyl | |
| 52 | 3α-(6ß,7ß-Epoxy)-tropanyl methobromid | Cyclopentyl | |

Anmerkung: Alle Verbindungen der Tabelle schmelzen unter Zersetzung.

Tabelle III

Verbindungen der Formel

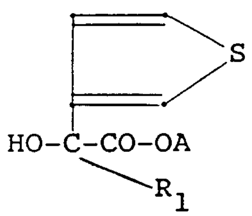

| Nr. | A | $R_1$ | Fp.[°C] (Hydrochlorid) |
|---|---|---|---|
| 1 | 3α-(6ß,7ß-Epoxy)-tropanyl | Phenyl | 246-7 |
| 2 | 3α-(6,7-Dehydro)-tropanyl | Phenyl | 261-2 |
| 3 | 3α-(6ß,7ß-Epoxy)-tropanyl | 3-Thienyl | |
| 4 | 3α-(6,7-Dehydro)-tropanyl | 3-Thienyl | |
| 5 | 3α-Tropanyl | 3-Thienyl | |
| 6 | 3α-(N-Methyl)-granatanyl | 3-Thienyl | |

## Tabelle IV

Verbindungen der Formel

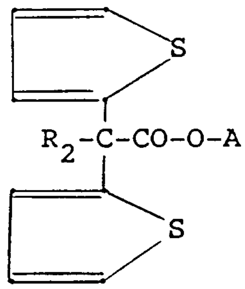

| Nr. | A | $R_2$ | Fp. [°C] (Hydrochlorid) |
|---|---|---|---|
| 1 | 3α-(6ß,7ß-Epoxy)-tropanyl | H | |
| 2 | 3α-(6,7-Dehydro)-tropanyl | H | |
| 3 | 3α-(6ß,7ß-Epoxy)-tropanyl | Methyl | |
| 4 | 3α-(6,7-Dehydro)-tropanyl | Methyl | 210-2,5 |
| 5 | 3α-(6ß,7ß-Epoxy)-tropanyl | Methoxy | |
| 6 | 3α-(6,7-Dehydro)-tropanyl | Methoxy | |

## Tabelle V

Verbindungen der Formel

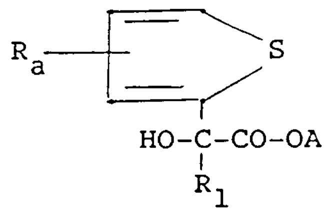

| Nr. | A | $R_1$ | $R_a$ | Fp. [°C] |
|---|---|---|---|---|
| 1 | 3α-(6ß,7ß-Epoxy)-tropanyl | 2-Thienyl | 5-Methyl | |
| 2 | 3α-(6,7-Dehydro)-tropanyl | 2-Thienyl | 5-Methyl | |
| 3 | 3α-Tropanyl | 2-Thienyl | 5-Methyl | |
| 4 | 3α-(6ß,7ß-Epoxy)-tropanyl | 2-(5-Methyl)-thienyl | 5-Methyl | |
| 5 | 3α-(6,7-Dehydro)-tropanyl | 2-(5-Methyl)-thienyl | 5-Methyl | |
| 6 | 3α-Tropanyl | 2-(5-Methyl)-thienyl | 5-Methyl | |
| 7 | 3α-(6ß,7ß-Epoxy)-tropanyl | 2-Thienyl | 5-Fluor | |
| 8 | 3α-(6,7-Dehydro)-tropanyl | 2-Thienyl | 5-Fluor | |
| 9 | 3α-Tropanyl | 2-Thienyl | 5-Fluor | |
| 10 | 3α-(6ß,7ß-Epoxy)-tropanyl | 2-(5-Fluor)-thienyl | 5-Fluor | |
| 11 | 3α-(6,7-Dehydro)-tropanyl | 2-(5-Fluor)-thienyl | 5-Fluor | |
| 12 | 3α-Tropanyl | 2-(5-Fluor)-thienyl | 5-Fluor | |

Tabelle VI

Quartärverbindungen der Formel

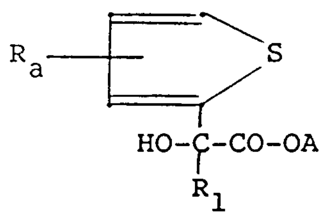

| Nr. | A | $R_1$ | $R_a$ | Fp.[°C] |
|---|---|---|---|---|
| 1 | 3α-(6ß,7ß-Epoxy)-tropanyl-methobromid | 2-Thienyl | 5-Methyl | |
| 2 | 3α-(6,7-Dehydro)-tropanyl-methobromid | 2-Thienyl | 5-Methyl | |
| 3 | 3α-Tropanyl-methobromid | 2-Thienyl | 5-Methyl | |
| 4 | 3α-(6ß,7ß-Epoxy)-tropanyl-methobromid | 2-(5-Methyl)-thienyl | 5-Methyl | |
| 5 | 3α-(6,7-Dehydro)-tropanyl-methobromid | 2-(5-Methyl)-thienyl | 5-Methyl | |
| 6 | 3α-Tropanyl-methobromid | 2-(5-Methyl)-thienyl | 5-Methyl | |
| 7 | 3α-(6ß,7ß-Epoxy)-tropanyl-methobromid | 2-Thienyl | 5-Fluor | |
| 8 | 3α-(6,7-Dehydro)-tropanyl-methobromid | 2-Thienyl | 5-Fluor | |
| 9 | 3α-Tropanyl-methobromid | 2-Thienyl | 5-Fluor | |
| 10 | 3α-(6ß,7ß-Epoxy)-tropanyl-methobromid | 2-(5-Fluor)-thienyl | 5-Fluor | |
| 11 | 3α-(6,7-Dehydro)-tropanyl-methobromid | 2-(5-Fluor)-thienyl | 5-Fluor | |
| 12 | 3α-Tropanyl-methobromid | 2-(5-Fluor)-thienyl | 5-Fluor | |

Tabelle VII

Verbindungen der Formel

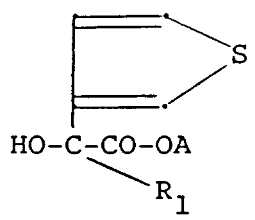

| Nr. | A | $R_1$ | Fp.[°C] |
|---|---|---|---|
| 1 | 3α-(6ß,7ß-Epoxy)-tropanyl methobromid | Phenyl | 211-2 |
| 2 | 3α-(6,7-Dehydro)-tropanyl methobromid | Phenyl | 158-60* |
| 3 | 3α-(6ß,7ß-Epoxy)-tropanyl methobromid | 3-Thienyl | |
| 4 | 3α-(6,7-Dehydro)-tropanyl methobromid | 3-Thienyl | |
| 5 | 3α-Tropanyl methobromid | 3-Thienyl | |
| 6 | 3α-(N-Methyl)-granatanyl methobromid | 3-Thienyl | |

* (mit Kristallmethanol)

Tabelle VIII

Quartärverbindungen der Formel

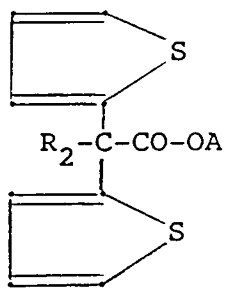

| Nr. | A | $R_2$ | Fp.[°C] |
|---|---|---|---|
| 1 | 3α-(6ß,7ß-Epoxy)-tropanyl methobromid | H | |
| 2 | 3α-(6,7-Dehydro)-tropanyl methobromid | H | |
| 3 | 3α-(6ß,7ß-Epoxy)-tropanyl methobromid | Methyl | |
| 4 | 3α-(6,7-Dehydro)-tropanyl methobromid | Methyl | 206-8 |
| 5 | 3α-Tropanyl methobromid | Methoxy | |
| 6 | 3α-(N-Methyl)-tropanyl methobromid | Methoxy | |

**Patentansprüche**

**1.** Verbindungen der Formel

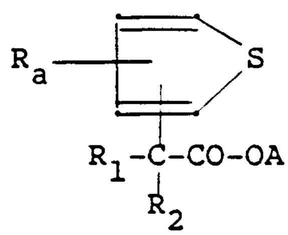

(I),

in der

A für die Gruppe

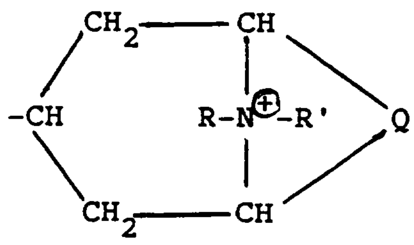

(II)

steht,
worin
Q eine der zweibindigen Gruppen
$-CH_2-CH_2-$, $-CH_2-CH_2-CH_2-$, $-CH=CH-$,

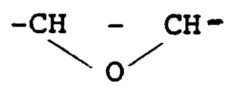

und
R einen gegebenenfalls halogen- oder hydroxysubstituierten $C_1$-$C_4$-Alkylrest,
R' einen $C_1$-$C_4$-Alkylrest bedeutet und R und R' gemeinsam auch einen $C_4$-$C_6$-Alkylenrest bilden können, und
der positiven Ladung des N-Atoms ein Äquivalent eines Anions gegenübersteht ($X^{\ominus}$),

$R_1$ für einen Thienyl-, Phenyl-, Furyl-, Cyclopentyl- oder Cyclohexylrest, wobei diese Reste auch methylsubstituiert, Thienyl und Phenyl auch fluor- oder chlorsubstituiert sein können,

$R_2$ für Wasserstoff, OH, $C_1C_4$-Alkoxy oder $C_1$-$C_4$-Alkyl,

$R_a$ für H, F, Cl oder $CH_3$ steht.

**2.** Verbindungen nach Anspruch 1, worin $R_1$ für 2-Thienyl steht.

**3.** Verbindungen nach Anspruch 1 oder 2, worin $R_2$ für OH steht.

**4.** Verbindungen nach Anspruch 1, 2 oder 3, worin A für

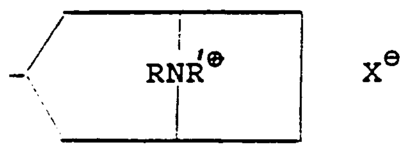

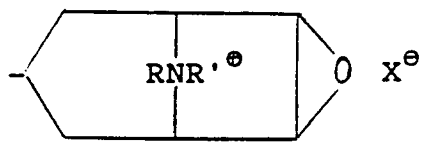

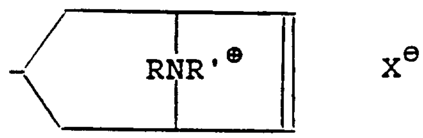

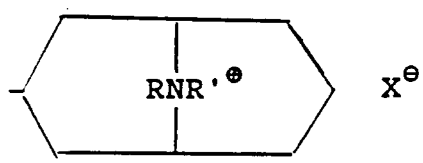

steht, R und $X^{\ominus}$ die obige Bedeutung haben und R' die obige Bedeutung ausgenommen Wasserstoff hat.

**5.** Verbindungen nach Anspruch 1 bis 4, in denen $R_1$ 2-Thienyl bedeutet und A für den Rest

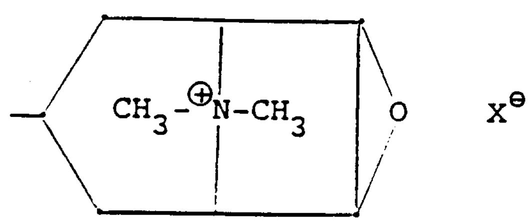

oder

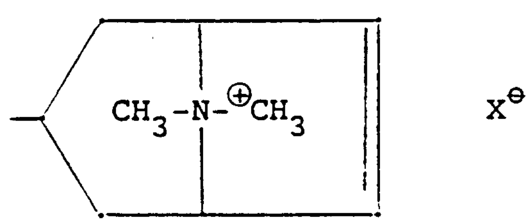

in der 3α-Form steht, worin $X^{\ominus}$ ein Äquivalent eines Anions ist, vorzugsweise $Br^{\ominus}$ oder $CH_3SO_3^{\ominus}$ .

**6.** Verbindungen der Formel

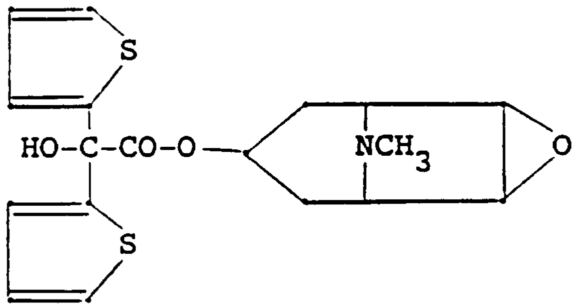

bzw.

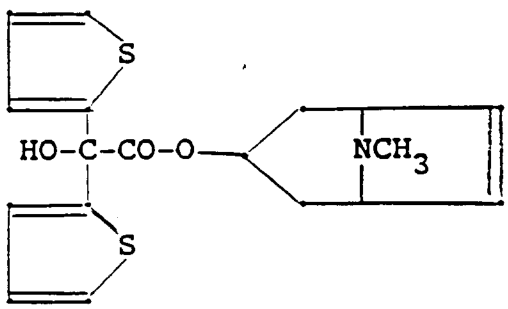

in der 3α-Form als Methobromid oder Methomethansulfonat.

**7.** Arzneimittel, gekennzeichnet durch einen Gehalt an einer Verbindung nach Anspruch 1 bis 6 neben üblichen Hilfs- und/oder Trägerstoffen.

**8.** Verwendung von Verbindungen nach Anspruch 1 bis 6 bei der Herstellung von anticholinergen Arzneimitteln.

**9.** Verwendung von Verbindungen nach Anspruch 1 bis 6 bei der Herstellung von Arzneimitteln zur Behandlung von Atemwegserkrankungen und Sinusbradykardien.

**10.** Verfahren zur Herstellung der Verbindungen nach Anspruch 1 bis 6, dadurch gekennzeichnet, daß man einen Ester der Formel

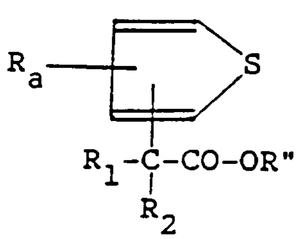

(IV),

worin R" für einen $C_1$-$C_4$-Alkylrest steht und $R_1$, $R_2$ und $R_a$ die obige Bedeutung haben, mit einem Aminoalkohol der Formel

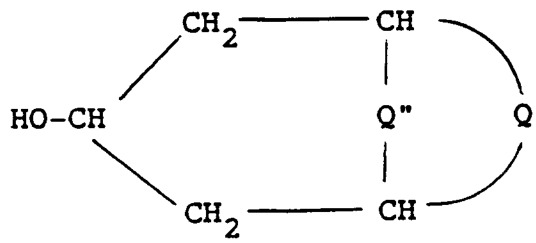

(V)

worin

Q die obige Bedeutung hat und Q" für =NR oder für =NH steht, in einem inerten organischen Lösungsmittel oder in der Schmelze in Gegenwart eines Umesterungskatalysators umestert und die erhaltene Verbindung der Formel

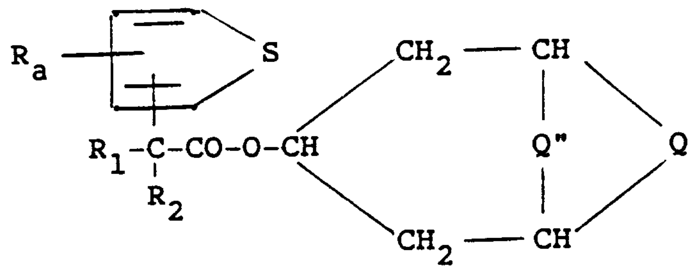

(VI)

a) wenn Q" =NR bedeutet, mit einem reaktionsfähigen Monoderivat Z-($C_1$-$C_4$-Alkyl) eines Alkans (Z Abgangsgruppe) quaterniert

oder

b) wenn Q" =NH bedeutet, mit einem endständig disubstituierten Alkan Z-($C_4$-$C_6$-Alkylen)-Z ohne Zwischenisolierung substituiert und quarterniert.

**11.** Verbindungen der Formel VI, in der Q, Q", $R_a$, $R_1$ und $R_2$ die obige Bedeutung haben und ihre Säureadditionssalze, mit der Maßgabe, daß die Tropinester der Phenyl-2-thienylglykolsäure, der 2,2'-Dithienylglykolsäure und der 3,3'-Dithienylglykolsäure ausgenommen werden.

**12.** Verbindungen nach Anspruch 11, worin Formel VI für

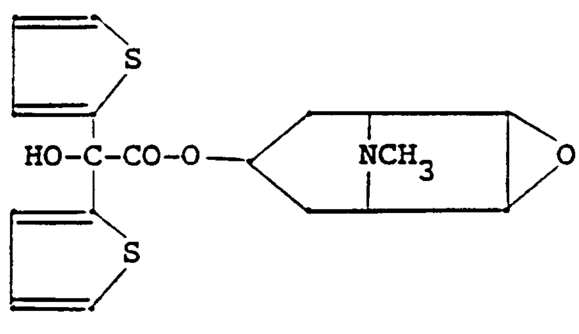

bzw.

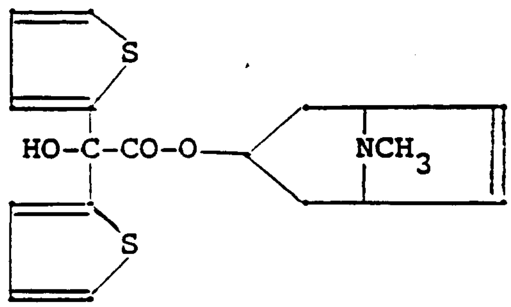

steht.

## Claims

**1.** Compounds of formula

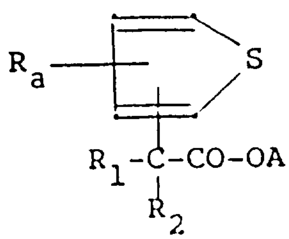

(I)

in which

A represents the group

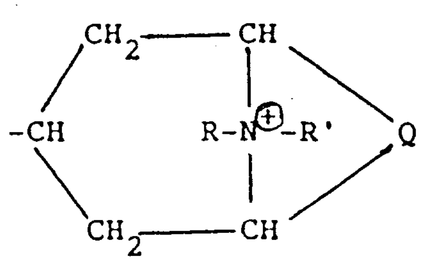

(II)

wherein

Q represents one of the double-bonding groups $-CH_2-CH_2-$, $-CH_2-CH_2-CH_2-$, $-CH=CH-$,

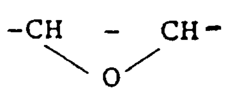

and R denotes an optionally halogen-substituted or hydroxy-substituted $C_1$-$C_4$ alkyl radical, R' denotes a $C_1$-$C_4$ alkyl radical and R and R' together may also form a $C_4$-$C_6$ alkylene radical and one equivalent of an anion ($X^{\ominus}$) opposes the positive charge of the N-atom,

$R_1$ represents a thienyl, phenyl, furyl, cyclopentyl or cyclohexyl radical, whereby these radicals may also be methyl-substituted, thienyl and phenyl may also be fluoro-substituted or chloro-substituted,

$R_2$ represents hydrogen, OH, $C_1$-$C_4$-alkoxy or $C_1$-$C_4$ alkyl,

$R_a$ represents H, F, Cl or $CH_3$.

**2.** Compounds according to claim 1, wherein $R_1$ represents 2-thienyl.

**3.** Compounds according to either claim 1 or 2, wherein $R_2$ represents OH.

**4.** Compounds according to claim 1, 2 or 3, wherein A represents

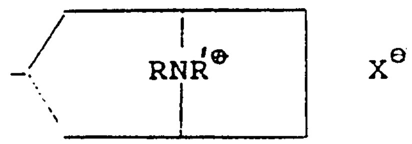

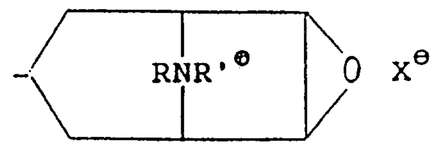

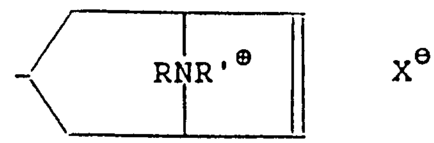

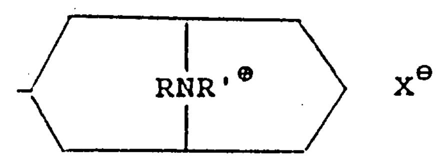

R and $X^{\ominus}$ have the above meaning and R' has the above meaning with the exclusion of hydrogen.

**5.** Compounds according to claims 1 to 4, in which $R_1$ denotes 2-thienyl and A represents the group

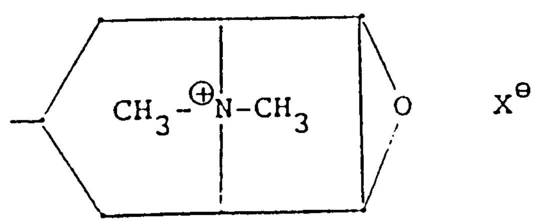

or

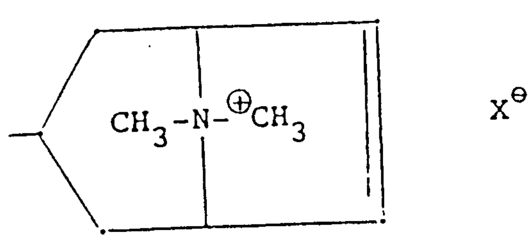

in the 3α-form, wherein $X^{\ominus}$ is one equivalent of an anion, preferably $Br^{\ominus}$ or $CH_3SO^{\ominus}_3$.

6. Compounds of Formula

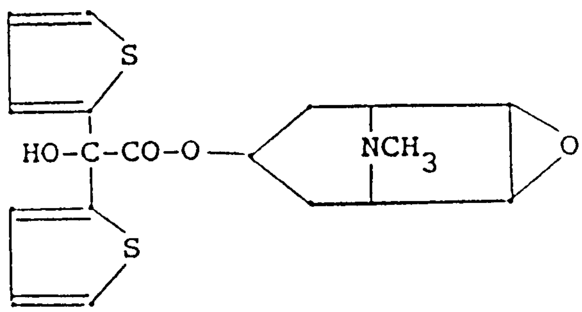

or

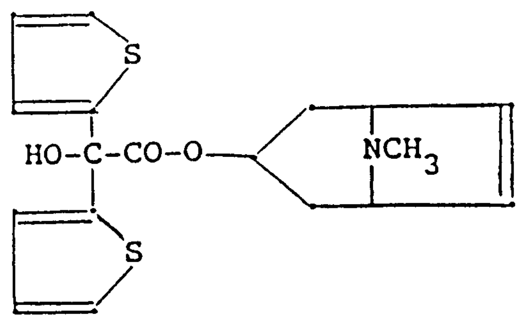

in the 3α-form as methobromides or methomethanesulfonates.

7. Medicaments characterised by an amount of a compound according to claims 1 to 6 in addition to conventional auxiliaries and/or excipients.

8. Use of compounds according to claims 1 to 6 for preparing anticholinergic medicaments.

9. Use of compounds according to claims 1 to 6 for preparing medicaments for the treatment of respiratory tract diseases and sinus bradycardia.

10. A process for preparing the compounds according to claims 1 to 6, characterised in that an ester of Formula

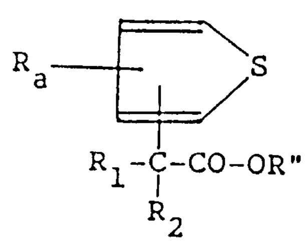

(IV)

wherein R" represents a $C_1$-$C_4$ alkyl radical and $R_1$, $R_2$ and $R_a$ have the above meaning is transesterified using an amino alcohol of Formula

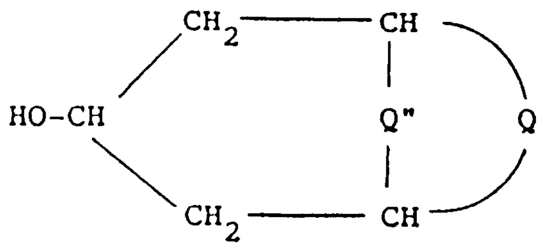

(V)

wherein
Q has the above meaning and Q" represents =NR or =NH, in an inert organic solvent or in the melt, in the presence of a transesterification catalyst, and the resultant compound of Formula

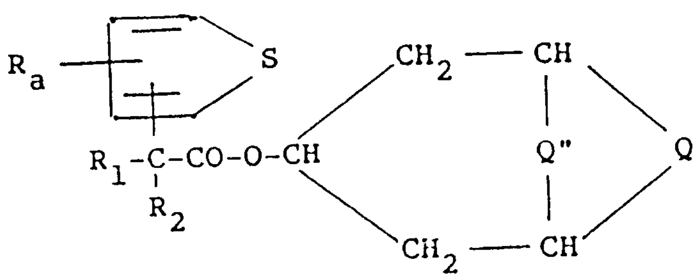

(VI)

a) is quaternised using a reactive monoderivative Z-($C_1$-$C_4$-alkyl) of an alkane (Z = leaving group), if Q" denotes =NR

or

b) is substituted and quaternised using a terminally disubstituted alkane Z-($C_4$-$C_6$-alkylene)-Z without intermediate isolation, if Q" denotes =NH.

**11.** Compounds of Formula VI, in which Q, Q", $R_a$, $R_1$ and $R_2$ have the above meaning, and their acid additions salts thereof with the proviso that the tropine esters of phenyl-2-thienylglycolic acid, 2,2'-dithienylglycolic acid and 3,3'-dithienylglycolic acid are excluded.

**12.** Compounds according to Claim 11, wherein Formula VI represents

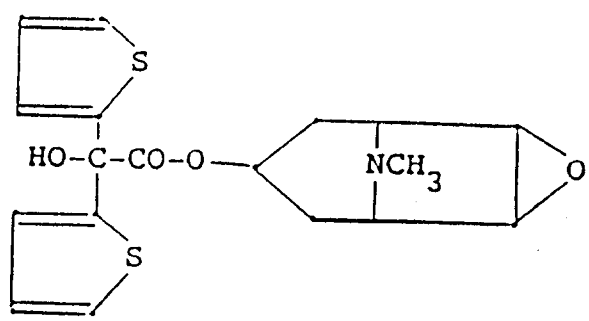

or

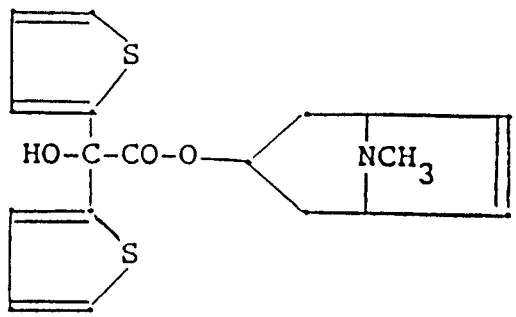

## Revendications

**1.** Composés de formule

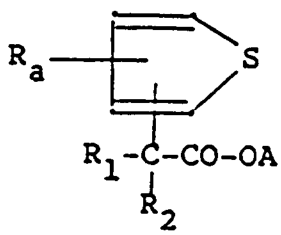

(I)

dans laquelle
A représente le groupe

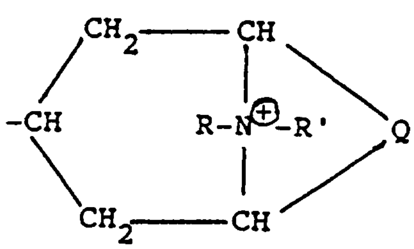

(II)

où
Q représente l'un des groupes divalents
$-CH_2-CH_2-$, $-CH_2-CH_2-CH_2-$, $-CH=CH-$,

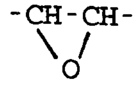

et
R représente un reste alkyle en $C_1$-$C_4$ éventuellement halogéno- ou hydroxysubstitué, R' représente un reste alkyle en $C_1$-$C_4$ et R et R' peuvent aussi former ensemble un reste alkylène en $C_4$-$C_6$, et un équivalent d'un anion ($X^-$) est opposé à la charge positive de l'atome N,
$R_1$ représente un reste thiényle, phényle, furyle, cyclopentyle ou cyclohexyle, ces restes pouvant aussi être méthylsubstitués, et les restes thiényle et phényle pouvant aussi être fluoro- ou chlorosubstitués,
$R_2$ représente l'hydrogène, OH, un groupe alcoxy en $C_1$-$C_4$ ou un groupe alkyle en $C_1$-$C_4$,
$R_a$ représente H, F, Cl ou $CH_3$.

**2.** Composés selon la revendication 1, dans lesquels $R_1$ représente un groupe 2-thiényle.

**3.** Composés selon la revendication 1 ou 2, dans lesquels $R_2$ représente OH.

**4.** Composés selon la revendication 1, 2 ou 3, dans lesquels A représente

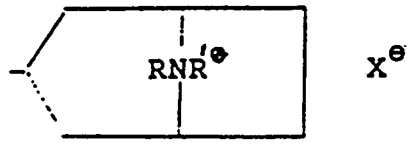

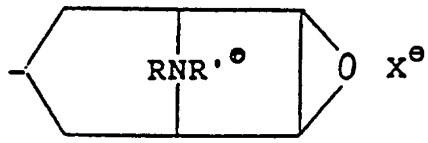

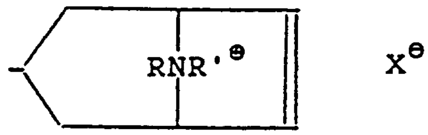

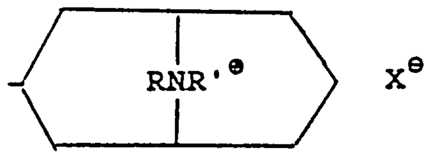

R et $X^-$ ont la signification ci-dessus et R' a la signification ci-dessus à l'exception de l'hydrogène.

**5.** Composés selon les revendications 1 à 4, dans lesquels $R_1$ représente un groupe 2-thiényle et A représente le reste

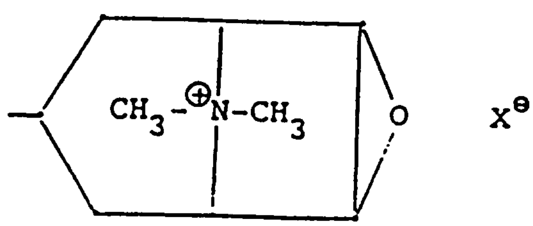

ou

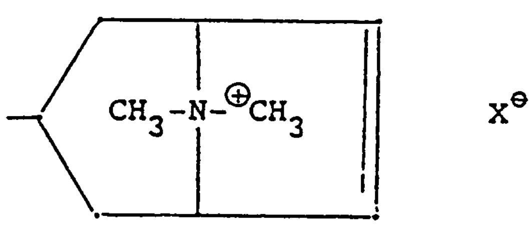

sous la forme 3α, où $X^-$ est un équivalent d'un anion, de préférence $Br^-$ ou $CH_3SO_3^-$.

**6.** Composés de formule

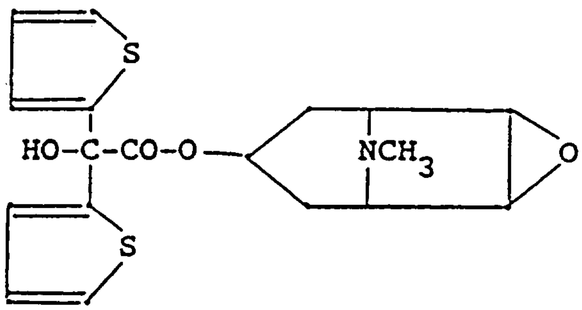

ou

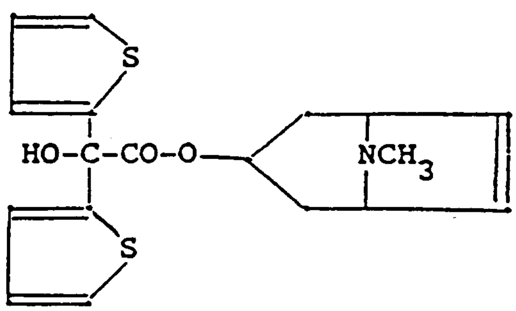

sous la forme 3α sous forme de méthobromure ou de méthométhanesulfonate.

**7.** Médicament caractérisé en ce qu'il contient un composé selon les revendications 1 à 6 outre des adjuvants et/ou véhicules courants.

**8.** Utilisation de composés selon les revendications 1 à 6 pour la préparation de médicaments anticholinergiques.

**9.** Utilisation de composés selon les revendications 1 à 6 pour la préparation de médicaments pour le traitement des maladies des voies respiratoires et des bradycardies sinusales.

**10.** Procédé de préparation des composés selon les revendications 1 à 6, caractérisé en ce que l'on transestérifie un ester de formule

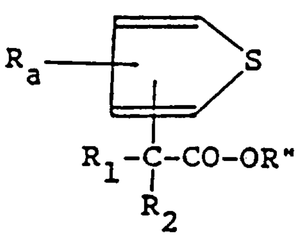

(IV)

dans laquelle R" représente un reste alkyle en $C_1$-$C_4$ et $R_1$, $R_2$ et $R_a$ ont la signification ci-dessus, avec un aminoalcool de formule

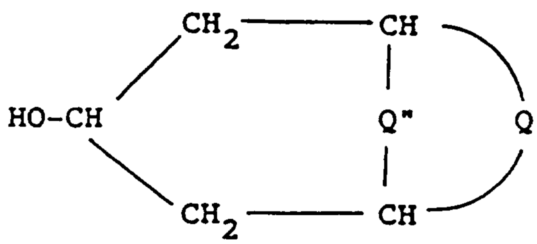

(V)

dans laquelle
Q a la signification ci-dessus et Q'' représente =NR ou =NH, dans un solvant organique inerte ou à l'état fondu, en présence d'un catalyseur de transestérification, et

a) lorsque Q'' représente =NR, on quaternise le composé obtenu de formule

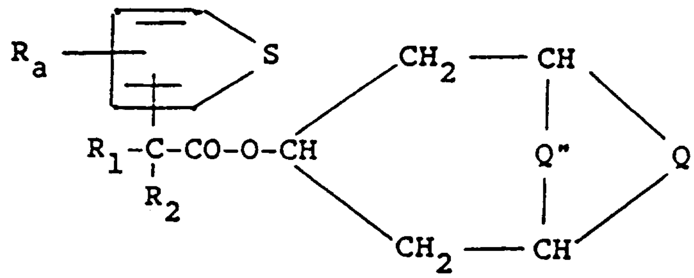

(VI)

avec un monodérivé réactif Z-(alkyle en $C_1$-$C_4$) d'un alcane (Z groupe partant)

ou

b) lorsque Q'' représente =NH, on substitue et quaternise le composé avec un alcane disubstitué aux extrémités Z-(alkylène en $C_4$-$C_6$)-Z sans isolement intermédiaire.

**11.** Composés de formule VI où Q, Q'', $R_a$, $R_1$ et $R_2$ ont la signification ci-dessus et leurs sels d'addition d'acide, à condition que les tropinesters de l'acide phényl-2-thiénylglycolique, de l'acide 2,2'-dithiénylglycolique et de l'acide 3,3'-dithiénylglycolique soient exclus.

**12.** Composés selon la revendication 11, dans lesquels la formule VI représente

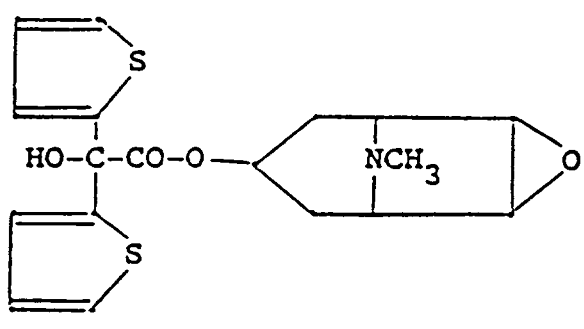

ou